# EUROPÄISCHE PATENTANMELDUNG

(11) **EP 2 801 326 A1**
(43) Veröffentlichungstag der Anmeldung: **12.11.2014**
(21) Anmeldenummer: 13166569.7
(22) Anmeldetag: 05.05.2013
(51) Int. Cl.: A61B 17/06, A61B 17/04

(54) **Wundnadel mit lösbarem Spitzenkörper und innenverlaufendem Faden**

(71) Anmelder: Doci Innovations GmbH, 23730 Sierksdorf (DE)
(72) Erfinder: Doci, Violeta, 22049 Hambourg (DE)
(74) Vertreter: Simandi, Claus

(57) **Zusammenfassung**

Die vorliegende Erfindung betrifft eine Wundnadel (10), welche in einer medizinischen Anwendung zum Nähen von Wunden genutzt werden kann. Es geht genauer um eine Wundnadel, welche mit einem Faden (20) verbunden ist und welche einen Spitzenkörper (22), der die Nadelspitze trägt, und einen Nadelkörper (12) aufweist, wobei der Nadelkörper (12) helixförmig ausgebildet ist und einen inneren Hohlraum aufweist, und der Nadelkörper ein erstes Ende (16) aufweist, das dem Spitzenkörper (22) zugewandet ist, und der Nadelkörper ein zweites Ende (18) aufweist, das dem ersten Ende (16) gegenüberliegt, und der Spitzenkörper (22) eine Fadenaufnahme aufweist und der Faden von der Fadenaufnahme vom ersten Ende (16) des Nadelkörpers durch den Hohlraum zum zweiten Ende (18) den Nadelkörpers geführt wird, dadurch gekennzeichnet, dass der Spitzenkörper als separates Bauteil ausgeführt ist, welches lösbar mit dem Nadelkörper verbunden ist, und Spitzenkörper, Schaftstück (24) und Verbindungsstück (26) aufweist und rotationssymmetrisch um eine Achse durch die Nadelspitze ausgeführt ist.

## Beschreibung

### Gebiet der Erfindung

Die vorliegende Erfindung betrifft eine Wundnadel, welche in einer medizinischen Anwendung zum Nähen von Wunden genutzt werden kann.

### Hintergrund der Erfindung

Das Nähen von Wunden zur Einleitung der Wundheilung ist eine alte Technik. Dementsprechend gibt es für verschiedene Anwendungen eine große Vielzahl von Varianten. Das typische Handwerkszeug des Arztes bleiben Nadel und Faden. Beides gibt es in reicher Auswahl und kann für die jeweilige Anwendung abgestimmt werden. Typischerweise werden auch fertige Nadel-/Fadenkombinationen im Handel angeboten.

Das europäische Patent EP 1 726 317 B1 offenbart chirurgische Mittel und ein chirurgisches Verfahren, welches sich insbesondere für kosmetische Operationen eignen soll. Dabei wird ein Faden in mehreren Spiralen, insgesamt ungefähr in Form einer Helix, in das Gewebe geführt. Dieses wird mit einer geraden Nadel erreicht.

Das europäische Patent EP 1 778 096 B1 offenbart eine spiralförmige Nähvorrichtung. Diese Nähvorrichtung ist speziell für Anwendungen im Bandscheibenbereich gedacht. Die Vorrichtung weist eine Nadel in Form einer Helix auf. Die Nadel ist innen hohl und kann im Hohlraum einen Faden führen. Am Ende der helixförmigen Nadel, also nahe ihrer Spitze, ist der Faden als Schlaufe geführt. Es ist möglich, mit einem Instrument mit einem Greifhaken (einer Häkelnadel nicht unähnlich) in die Schlaufe des Fadens am vorderen Ende der helixförmigen Nadel einzugreifen und diesen Faden dann zu greifen. In dieser Weise lässt sich der Faden auf einer helixförmigen Bahn führen und anschließend verknoten. Anspruchsvoll in dem Verfahren ist offenkundig, dass zwei Instrumente benötigt werden, und der Faden in eine Position gebracht werden muss, wo er mit dem Haken gut gegriffen werden kann.

Das US-Patent US 5,356,424 offenbart eine Nadel-/Fadenkombination speziell für die minimal invasive Chirurgie. Dabei wird eine Nadel verwendet, welche ebenfalls einen helixförmigen Abschnitt umfasst. Diese Nadel ist innen hohl und führt einen Faden. Die Nadel sitzt auf einem Nadelhalter, welcher etwa die Form eines Stiftes hat. In diesen Nadelhalter wird die Nadel geführt. Sie ist aber lösbar mit dem Nadelhalter verbunden und kann auch abgenommen werden. Zu beachten ist, dass es sich bei dem Nadelhalter um eine spezielle auf die Nadel abgestimmte Konstruktion handelt, welche stark von der Bauart üblicher, in etwa pinzettenförmiger Nadelhalter abweicht. Eine Art der Anwendung dieses Gerätes besteht darin, den Faden am spitzen Ende aus der Nadel zu ziehen. Es ist offenkundig, dass der Faden dabei schwer zu führen ist und leicht reißen kann.

Die deutsche Offenlegungsschrift DE 10 2008 044 855 offenbart ein Gerät und ein Verfahren zum chirurgischen Wundverschluß. Dabei kommt eine hohle Spiralnadel zum Einsatz. In der Nähe der Spitze weist die Spiralnadel eine Sollbruchstelle auf, an der die Spitze vom übrigen Nadelkörper getrennt werden kann. Die Spitze kann dann mit einer OP-Pinzette festgehalten werden.

Auf dem Gebiet der Fäden gibt es ebenfalls viele Entwicklungen und Verbesserungen. Moderne Fäden aus mehreren Komponenten offenbart beispielsweise die europäische Patentschrift EP 1 171 172 B1 und auch die europäische Patentschrift EP 1 293 218 B1, welche ein mutmaßlich besonders starkes Fadenmaterial offenbaren.

Die vorliegende Erfindung strebt es an, diesen Stand der Technik zu verbessern. Insbesondere soll eine Wundnadel zur Verfügung gestellt werden, mit der auch an weniger zugänglichen Stellen sicher Nähte gelegt werden können. Das Legen der Naht soll dabei einfach sein und der Faden soll in einer gut vorherbestimmbaren Weise verlaufen. Die Rückführung der Nadel aus dem Gewebe soll leicht und sicher sein und auch in schwierigen Situationen soll der richtige Sitz des Fadens während der Rückführung gewährleistet bleiben.

### Nähere Beschreibung der Erfindung

Diese Aufgabe löst eine Wundnadel nach Anspruch 1 und in korrespondierender Weise ein Verfahren nach Anspruch 13.

Bei der vorliegenden Erfindung geht es also um eine Wundnadel, welche nach Anspruch 1 als wichtige Elemente einen Spitzenkörper und einen Nadelkörper aufweist. Der Spitzenkörper trägt die Nadelspitze, ist also an dem spitzen Ende der Nadel positioniert. Der Spitzenkörper selbst weist im Anschluss an die Nadelspitze ein Schaftstück auf und ein Verbindungsstück. Mit diesem Verbindungsstück kann die Verbindung zum Nadelkörper hergestellt werden.

Der Nadelkörper selbst ist seinem Gewicht und seiner Größe nach in aller Regel der hauptsächliche Teil der Nadel. Im Sinne der vorliegenden Erfindung ist er helixförmig ausgebildet. Es kommt dabei nicht auf eine mathematisch exakt ausgebildete Helix an, sondern vielmehr darauf, dass der Nadelkörper in einer Projektionsebene rund oder annähernd kreisförmig oder elliptisch oder annähernd elliptisch ausgebildet ist und in einer dazu senkrechten Ebene so ausgebildet ist, dass sich durch Rotation im Gewebe ein Vorschub ergibt. Eine dazu geeignete Form ist eine Zig-Zag-Form mit dreidimensionaler Ausdehnung. Eine mathematisch exakte oder angenähert mathematische exakte Helix ist jedoch durchaus zweckmäßig.

Der helixförmig ausgebildete Nadelkörper weist einen inneren Hohlraum auf. In diesen inneren Hohlraum kann ein Faden geführt werden. Der Hohlraum weist ein erstes Ende auf, welches dem Spitzenkörper und damit der Nadelspitze zugewandt ist. Der Hohlraum weist auch ein zweites Ende auf, welches dem ersten Ende gegenüberliegt. Der Faden kann über das zweite Ende hinausragen.

Zur Führung des Fadens weist am anderen Ende, also in der Nähe des ersten Endes des Hohlkörpers, der Spitzenkörper eine Fadenaufnahme auf. Die Form der Fadenaufnahme ist nicht sehr entscheidend. Es käme im Prinzip ein klassisches Nadelöhr in Frage, es kommt aber auch eine Sicke in Frage, in die ein Fadenende bloß eingelegt wird. Der Faden kann auch auf eine Führungsfläche geklebt werden oder um einen Teil des Spitzenkörpers umwickelt werden. In der Regel wird die Wundnadel fertig mit einem Faden ausgeliefert. Daher kann die Fadenaufnahme so gestaltet sein, dass sie nur für einen Faden geeignet ist.

Im Sinne der vorliegenden Erfindung ist der Spitzenkörper als separates Bauteil ausgeführt. Er soll lösbar mit dem Nadelkörper verbunden sein. Der Spitzenkörper soll eine Nadelspitze, ein Schaftstück und ein Verbindungsstück aufweisen. Dieses Verbindungsstück dient zur Verbindung des Nadelkörpers. Ferner soll der Spitzenkörper rotationssymmetrisch um eine Achse durch die Nadelspitze ausgeführt sein.

Es hat sich gezeigt, dass die rotationssymmetrische Ausführung gerade bei der Versorgung sehr kleiner Wunden hilfreich ist. Sie ist insbesondere in Kombination mit dem helixförmigen Nadelkörper hilfreich. Aufgrund der Helixform des Nadelkörpers wird nicht in direkter Weise Druck auf die Nadelspitze ausgeübt. Der Druck kommt im Wesentlichen durch eine Drehbewegung am anderen Ende der Helix zustande, es gibt also anders als bei einer geraden Nadel keine gerade Linie vom Druckausübungspunkt auf die Nadelspitze. Im Übrigen kann es leicht sein, dass sich die Helix, welche ja als Hohlraum und häufig als dünnwandiger Hohlraum ausgeführt ist, unter Druck verzieht.

Unter diesen Umständen wäre es grundsätzlich gut denkbar, dass die Nadelspitze einen wenig vorhersehbaren Winkel zum Gewebe annimmt und der Druckpunkt ebenfalls nicht ideal ist. In solcher Weise kann das Durchdringen des Gewebes schwerer sein als erforderlich und dabei können im Gewebe mehr Schäden verursacht werden, als es eigentlich erforderlich wäre.

Eine typische einfache Nadelspitze zeigt beispielsweise das schon zitierte europäische Patent EP 1 778 096 B1. Dort ist eine typische Spitzenform gezeigt, wie sie durch einen bloßen Schrägschnitt durch ein Röhrenende erzeugt wird. Wie ausgeführt, ist diese Spitzenform gerade beim Setzen einer Wundnaht mit sehr kleiner Dimension, beispielsweise bei einer Augenoperation, nicht ideal.

Die rotationssymmetrische Ausführung des Schaftstückes um eine Achse durch die Nadelspitze sorgt dafür, dass das Schaftstück Druck gut aufnehmen kann und in vorhersehbarer und kontrollierter Weise an die Nadelspitze abgibt. Hierbei entstehen keine großen Querkräfte, so dass die Nadelspitze auf ihrem Sollpfad verbleibt.

Es ist zweckmäßig, wenn am Nadelkörper ein Lager vorgesehen ist, durch welches Druck auf den Spitzenkörper weitergegeben werden kann. Ein solches Lager führt dazu, dass eine definierte Druckweitergabe erfolgt. Zweckmäßig ist insbesondere, wenn das Lager eine umlaufende Stützfläche aufweist. Eine umlaufende Stützfläche kann dabei um 360 Grad um die Achse durch die Nadelspitze umlaufen. Es kann auch genügen, eine umlaufende, aber unterbrochene Stützfläche vorzusehen, welche beispielsweise um die Achse und um die Nadelspitze umlaufend nur drei 100°-Segmente oder vier 60°-Segmente abdeckt, oderdergleichen. Die Stützfläche kann dabei eine Endfläche (beispielsweise eine Schnittfläche) oder ein Vorsprung sein.

Vorteilhaft ist ebenfalls, wenn für das Verbindungsstück des Spitzenstückes im Nadelkörper eine formflüssige Seitenführung vorgesehen ist. Eine solche formflüssige Seitenführung verhindert das Verkanten des Spitzenstückes gegenüber dem Nadelkörper. Das Verbindungsstück kann beispielsweise als Rohrstück ausgeführt sein und der Nadelkörper kann mit einem Rohrstück größeren Umfanges enden, welcher das Verbindungsstück passgenau aufnimmt. Eine formflüssige Seitenführung erlaubt es auch in einfacher Weise, den Spitzenkörper lösbar vom Nadelkörper zu gestalten.

Zweckmäßig ist ferner, wenn der Spitzenkörper bündig in den Nadelkörper übergeht. In diese Weise ergibt sich an der Außenfläche ein glatter Übergang zwischen Spitze und Nadel. Dadurch wird unnötiger Schaden am Gewebe vermieden.

Am Spitzenkörper kann ferner eine Hemmung vorgesehen werden. Diese Hemmung dient dazu, das Ablösen des Spitzenkörpers vom Nadelkörper einzuleiten. Eine solche Hemmung soll einen Widerstand bei der Rückbewegung der Nadel bilden. Dazu ist mindestens ein ausklappbarer Stift zweckmäßig. Die Hemmung leitet das Ablösen des Spitzenkörpers vom Nadelkörper ein, dient aber nicht als einziger Wiederstand gegen eine Rückbewegung des Spitzenkörpers. Vielmehr kann dazu ein weiteres Bauteil vorgesehen sein, beispielsweise ein Spreitzfächer, das nach Ablösung des Spitzenkörpers vom Nadelkörper wirksam wird.

Besonders vorteilhaft kann es demnach auch sein, wenn am Spitzenkörper ein Spreitzfächer vorgesehen ist. Dieser Spreitzfächer verhindert das Zurückrutschen eines Fadens beim Rückführen der Nadel. Seine genauere Funktion wird aus den Anmerkungen zum Verfahren zum Setzen einer Wundnaht und aus den Zeichnungen deutlich. Vorteilhaft ist es dann ebenfalls, wenn im Nadelkörper ein Aufnahmeraum für den Spreitzfächer vorgesehen ist.

Zu beachten ist, dass das Vorsehen eines Spreitzfächers am Spitzenkörper grundsätzlich vorteilhaft ist und beispielsweise auch unabhängig von der Geometrie des Spitzenkörpers angestrebt werden kann. Daher ist bezieht sich die Erfindung auch auf eine Wundnadel, welche mit einem Faden verbunden ist und welche einen Spitzenkörper, der die Nadelspitze trägt, und einen Nadelkörper aufweist, wobei der Nadelkörper helixförmig ausgebildet ist und einen inneren Hohlraum aufweist, und der Nadelkörper ein erstes Ende aufweist, das dem Spitzenkörper zugewandet ist, und der Nadelkörper ein zweites Ende aufweist, das dem ersten Ende gegenüberliegt, und der Spitzenkörper eine Fadenaufnahme aufweist und der Faden von der Fadenaufnahme vom ersten Ende des Nadelkörpers durch den Hohlraum zum zweiten Ende den Nadelkörpers geführt wird, wobei der der Spitzenkörper als separates Bauteil ausgeführt ist, welches lösbar mit dem Nadelkörper verbunden ist, und Spitzenkörper, Schaftstück und Verbindungsstück aufweist und am Spitzenkörper ein Spreitzfächer vorgesehen ist.

Beim Legen sehr kleiner Wundennähte gibt es eine Präferenz für glatte Fäden. Solche glatten Fäden gelten als minimal gewebeschädigend. Eine entsprechende Präferenz für monofilamente Fäden ist auch in der deutschen Offenlegungsschrift DE 10 2008 044 855 festgehalten. Überraschenderweise hat sich allerdings im Rahmen der vorliegenden Erfindung die Erkenntnis durchgesetzt, dass auch ein mehrfasriger Faden sehr zweckmäßig ist. Vorteilhaft ist durchaus ein Faden mit rauer Oberfläche. Die Mehrfasrigkeit des Fadens und insbesondere die dadurch bewirkte raue Oberfläche führen nämlich zu einem geringen Reibungswiderstand zwischen dem Faden und der Wundnadel. Dies macht die Rückführung der Wundnadel besonders einfach. Ein besonders zweckmäßiger Faden ist dabei ein geflochtener oder gesponnener Faden.

Vorteilhaft ist eine Wundnadel, bei welcher sich die Spitze durch Zugkräfte von weniger als 1 Newton (N) oder sogar weniger als 0,5 N oder weniger als 0,25 N vom Nadelkörper lösen lässt.

Im Rahmen der vorliegenden Erfindung wurde erkannt, dass ein Verfahren folgender Art nützlich ist:

Verfahren zum Setzen einer Wundnaht zwischen einem ersten Wundgewebe und einem zweiten Wundgewebe, beim dem ein Faden und eine Wundnadel mit einem Nadelkörper und einer lösbaren Spitze, an welcher der Faden gehalten wird, verwendet werden, wobei das Verfahren folgende Schritte in der Reihenfolge ihrer Auflistung umfasst:
- Setzen eines Einstichs mit der Nadel im ersten Wundgewebe
- Weiterführen der Nadel zum zweiten Wundgewebe, wobei die Nadel durch den Einstich im ersten Wundgewebe geführt wird
- Setzen eines Einstichs im zweiten Wundgewebe, wobei die Nadel weiterhin durch den Einstich im ersten Wundgewebe geführt wird
- Führen der Spitze aus dem zweiten Wundgewebe, wobei die Nadel weiterhin durch den Einstich im ersten Wundgewebe geführt wird und wobei der Weg der Spitze der Nadel in den vorangegangenen Schritten eine Einführrichtung und einen Einführpfad festlegt
- Lösen der Nadelspitze vom Nadelkörper im Wesentlichen durch Ausüben von Zug entgegen der Einführrichtung
- Rückführen des Nadelkörpers ohne die Spitze aus dem zweiten Wundgewebe und aus dem ersten Wundgewebe in umgekehrter Richtung entlang des Einführpfades, wobei der Faden im Wundgewebe verbleibt

Ein solches Verfahren ist auch in den eher kommerziell geprägten Bereichen der Medizin nützlich, wie beispielsweise in der Schönheitschirurgie, welche auch Eingriffe ohne medizinische Notwendigkeit an gesunden Patienten umfasst.

Aus dem Verständnis dieses Verfahrens, sind viele Vorteile der Erfindung besonders deutlich erkennbar. Zu beachten ist auch, dass dieses Verfahren zur Anwendung am Menschen nützlich, aber auch in der veterinärmedizischen Anwendung, also zum Versorgen von Wunden bei Tieren.

Das Verfahren kann um einen Schritt ergänzt werden, in dem beim Rückführen des Nadelkörpers nur der Nadelkörper gehalten wird. Ferner kann sich beim Rückführen des Nadelkörpers nur die Nadelspitze mit einem Spreitzfächer am Wundgewebe oder an benachbartem Gewebe abstützt. Besonders dieses Verfahren ist für Wundgewebe an schwer zugänglichen Stellen besonders geeignet.

Daher ist es ein Anliegen der Erfindung, Material zum Ausführen eines solchen Verfahrens zur Verfügung zu stellen, insbesondere eine geeignete Wundnadel.

Weitere Merkmale, aber auch Vorteile der Erfindung, ergeben sich aus den nachfolgend aufgeführten Zeichnungen und der zugehörigen Beschreibung. In den Abbildungen und in den dazugehörigen Beschreibungen sind Merkmale der Erfindung in Kombination beschrieben. Diese Merkmale können allerdings auch in anderen Kombinationen von einem erfindungsgemäßen Gegenstand umfasst werden. Jedes offenbarte Merkmal ist also auch als in technisch sinnvollen Kombinationen mit anderen Merkmalen offenbart zu betrachten. Bei den (teilweise leicht vereinfachten, schematischen) Abbildungen zeigen:
- Fig. 1: zeigt in perspektivischer Ansicht eine Wundnadel nach der vorliegenden Erfindung;
- Fig. 2: zeigt ebenfalls in perspektivischer Ansicht eine Wundnadel nach der vorliegenden Erfindung, bei der der Spitzenkörper vom Nadelkörper abgelöst wurde;
- Fig. 3: zeigt in vergrößerter Ansicht ein Ende des Nadelkörpers und den Spitzenkörper einer Ausführungsform der vorliegenden Erfindung;
- Fig. 4: zeigt in vergrößerter Ansicht ein Ende des Nadelkörpers und den Spitzenkörper in einer Ausführung mit einem Spreitzfächer, so dass die vergrößerte Ansicht der Ausführungsform aus Fig. 2 entspricht.

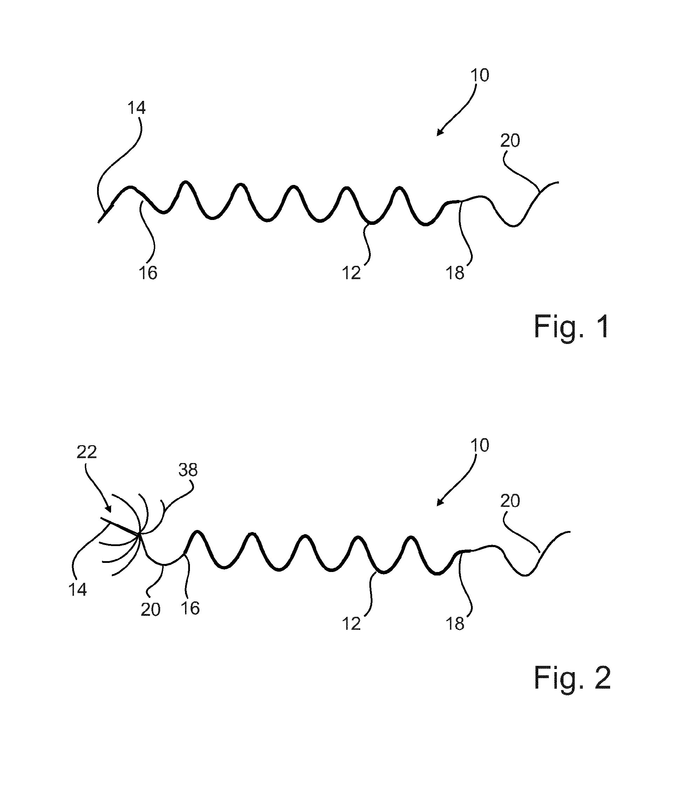

Fig. 1 zeigt die Wundnadel 10 in einer (darstellerisch leicht vereinfachten) perspektivischen Ansicht. Die Wundnadel 10 zeigt als wichtigen Bestandteil den Nadelkörper 12. Dieser hat die Form einer Helix. An einem Ende ist die Nadelspitze 14 ausgebildet. In der unmittelbaren Nachbarschaft der Nadelspitze 14 befindet sich das erste Ende 16 des Nadelkörpers 12. Ihm gegenüber liegt das zweite Ende 18 des Nadelkörpers 12. Aus dem zweiten Ende 18 ragt der Faden 20 heraus.

Die Wundnadel 10 ist also hier in dem Zustand vor oder während der Vernähung einer Wunde gezeigt. Die Nadelspitze 14 (welche auf einem hier noch nicht erkennbaren Spitzenkörper sitzt) ist in diesem Zustand fest mit dem Nadelkörper 12 verbunden.

Fig. 2 zeigt in entsprechender Ansicht eine erfindungsgemäße Wundnadel 10 in einem anderen Benutzungszustand. Erkennbar ist wieder der Nadelkörper 12 der Wundnadel 10 und ihr erstes Ende 16 sowie ihr zweites Ende 18.

Jedoch ist von dem Nadelkörper 12 der Spitzenkörper 22 abgelöst. Zwischen dem Spitzenkörper 22 ist der Faden 20 noch mit dem ersten Ende 16 des Nadelkörpers 12 verbunden. In dieser Ausführungsform weist der Spitzenkörper 22 einen Spreitzfächer 38 auf. Wie beschrieben, kann sich der Spreitzfächer 38 an einem Gewebeteil abstützen (und zwar in aller Regel, ohne dieses irgendwie zu beschädigen). Die Rückführung des Nadelkörpers 12 (im Wesentlichen) auf dem Einführpfad, jedoch in umgekehrter Richtung und somit entlang eines Rückführpfades, sorgt dafür, dass sich Nadelkörper 12 und der Spitzenkörper 22 immer weiter voneinander entfernen. Beide sind noch durch den Faden 20 verbunden. Der Faden 20 gleitet bei dieser Bewegung durch den Hohlraum des Nadelkörpers 12. Die Länge des Fadens 20, welche noch über das zweite Ende 18 des Nadelkörpers 12 hinausragt, verkürzt sich dementsprechend.

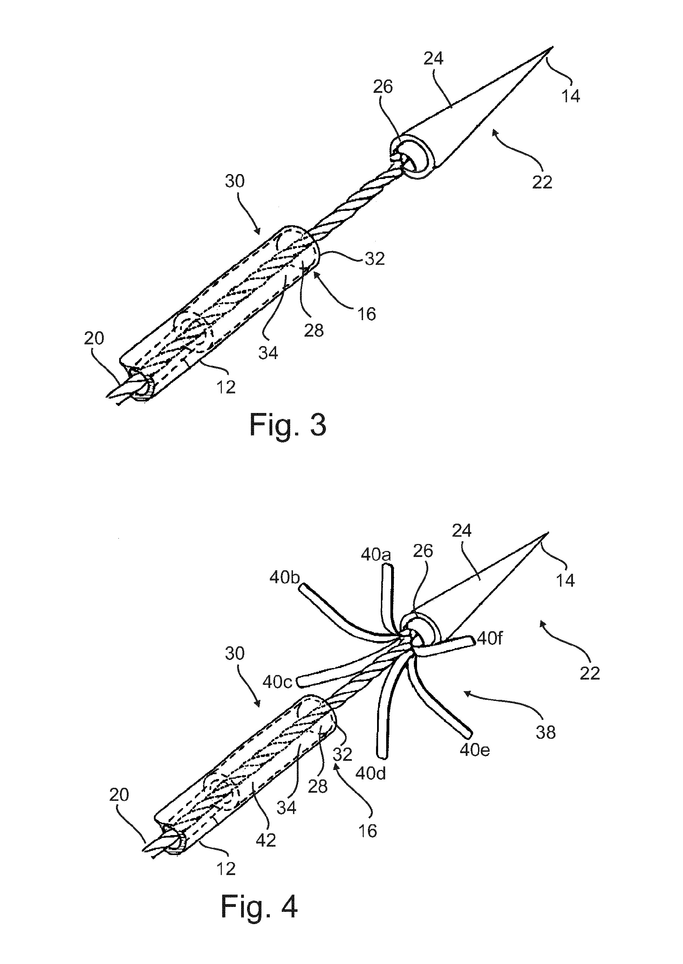

Fig. 3 zeigt in vergrößerter Ansicht einen Spitzenkörper 22 und den Nadelkörper 12 im Bereich seines ersten Endes 18. Fig. 3 zeigt diese Bauteile für eine Nadel nach der vorliegenden Erfindung, jedoch in anderer Ausführungsform als die in Fig. 2 gezeigte. Wie man in dieser Darstellung gut erkennt, weist der Spitzenkörper 22 vorne die Nadelspitze 14 auf und auf den Nadelkörper 12 zugewandt ein Verbindungsstück 26. Zwischen Verbindungsstück 26 und Nadelspitze 14 weist der Spitzenkörper 22 ein Schachtstück 24 auf. Man erkennt, dass der Spitzenkörper 22 rotationssymmetrisch um eine Hauptachse durch die Nadelspitze 14 ausgeführt ist.

Der Spitzenkörper 22 ist in einer nicht näher dargestellten Weise mit dem Faden 20 verbunden. Es handelt sich um einen mehrfasrigen Faden. Dieser Faden 20 verläuft im Hohlraum des Nadelkörpers 12, welcher hier nur im Bereich seines ersten Endes 16 dargestellt ist. Im Bereich des ersten Endes 16 ist der Hohlraum des Nadelkörpers 12 erweitert. Dies lässt sich beispielsweise durch eine Bohrung erreichen.

In diesem Bereich ist ein Lager 30 ausgebildet, welches eine Stützfläche 32 und eine Seitenführung 34 umfasst. Am ersten Ende 16 des Nadelkörpers 12 ist eine Endfläche ausgebildet, welche glatt ist und eine Stützfläche 32 darstellt. Diese Stützfläche 32 ist umlaufend um die Längsachse des Nadelkörpers 12 in diesem Endbereich gestaltet. Sie beschreibt also einen 360-Grad-Winkel um den Faden 20.

An dem ersten Ende 16 ist ferner ein Aufnahmeraum 28 für das Verbindungsstück 26 vorgesehen. Die röhrenförmige Gestaltung des Nadelkörpers 12 in diesem Bereich sorgt ferner bereits dafür, dass es eine Seitenführung 34 für das Verbindungsstück 26 gibt. Die Stützfläche 32 und die Seitenführung 34 bieten hier in dieser Weise insgesamt ein Lager 30 an, im welchem der Spitzenkörper 22, wie gezeigt vorzugsweise mit seinem Verbindungsstück 26, gelagert werden kann.

Fig. 4 zeigt in gleicher Ansicht wie Fig. 3 eine andere Ausführungsform der Nadelspitze 14. Die Grundelemente, welche für Fig. 3 beschrieben wurden, werden auch bei dieser Ausführungsform verwendet. Diese Ausführungsform weist jedoch zusätzlich einen Spreitzfächer 38 auf. Dieser Spreitzfächer 38 besteht aus sechs Spreitzlamellen 40a, 40b, 40c, 40d, 40e und 40f. Diese Lamellen sind fest mit dem Spitzenkörper 22 verbunden. Man erkennt in dieser Abbildung auch, dass der Aufnahmeraum 28 länger ausgebildet ist, als es zur Aufnahme des Verbindungsstückes 26 erforderlich ist. An dem Aufnahmeraum 28 schließt sich daher ein Aufnahmeraum 42 an, welcher die Spreitzlamellen 40 aufnehmen kann. Vor der Abtrennung des Spitzenkörpers 22 vom Nadelkörper 12 liegen die Lamellen in dem Aufnahmeraum 42. Dies erlaubt es, dass in der ersten Phase der Verwendung der Nadel, der Nadelkörper an seinem ersten Ende 16 bündig in den Spitzenköper 22 übergeht. Die Spreitzlamellen 40 sind dann vollständig in dem Aufnahmeraum 42 verborgen. Da die Lamellen sich selbständig aufspreitzen, drücken sie in diesem Zustand gegen die Innenwände des Aufnahmeraumes 42. Dies sorgt für eine noch festere Verbindung zwischen Spitzenkörper 22 und dem Nadelkörper 12.

Insgesamt zeigt sich, dass mit diesem Konzept neuartige Möglichkeiten zur verbesserten Vernähung von Wunden gegeben sind.

**Bezugszeichenliste**

| | |
|---|---|
| 10 | Wundnadel |
| 12 | Nadelkörper |
| 14 | Nadelspitze |
| 16 | erstes Ende |
| 18 | zweites Ende |
| 20 | Faden |
| 22 | Spitzenkörper |
| 24 | Schaftstück |
| 26 | Verbindungsstück |
| 28 | Aufnahmeraum |
| 30 | Lager |
| 32 | Lagerfläche /Druckfläche /Anstoßfläche |
| 34 | Seitenführung |
| 36 | Hemmung |
| 38 | Spreitzfächer |
| 40 | Spreitzlamellen |
| 42 | Aufnahmeraum für Spreitzfächer |

## Patentansprüche

1. Wundnadel (10), welche mit einem Faden (20) verbunden ist und welche einen Spitzenkörper (22), der die Nadelspitze trägt, und einen Nadelkörper (12) aufweist, wobei der Nadelkörper (12) helixförmig ausgebildet ist und einen inneren Hohlraum aufweist, und der Nadelkörper (12) ein erstes Ende (16) aufweist, das dem Spitzenkörper (22) zugewandet ist, und der Nadelkörper (12) ein zweites Ende (18) aufweist, das dem ersten Ende gegenüberliegt, und der Spitzenkörper (22) eine Fadenaufnahme aufweist und der Faden (20) von der Fadenaufnahme vom ersten Ende des Nadelkörpers (12) durch den Hohlraum zum zweiten Ende den Nadelkörpers (12) geführt wird, **dadurch gekennzeichnet, dass** der Spitzenkörper (22) als separates Bauteil ausgeführt ist, welches lösbar mit dem Nadelkörper (12) verbunden ist, und Spitzenkörper (22), Schaftstück (24) und Verbindungsstück (26) aufweist und rotationssymmetrisch um eine Achse durch die Nadelspitze ausgeführt ist.

2. Wundnadel (10) nach Anspruch 1, bei welchem am Nadelkörper (12) ein Lager (30) vorgesehen ist, durch welches Druck auf den Spitzenkörper (22) weitergegeben werden kann.

3. Wundnadel (10) nach Anspruch 2, bei welcher das Lager (30) einen umlaufenden Stützfläche (32) aufweist.

4. Wundnadel (10) nach einem der vorhergehenden Ansprüche, bei welchem für das Verbindungsstück (26) des Spitzenkörpers (22) im Nadelkörper (12) eine formschlüssige Seitenführung (34) vorgesehen ist.

5. Wundnadel (10) nach einem der vorhergehenden Ansprüche, bei welchem der Spitzenkörper (22) bündig in den Nadelkörper (12) übergeht.

6. Wundnadel (10) nach einem der vorhergehenden Ansprüche, bei welcher am Spitzenkörper (22) eine Hemmung vorgesehen ist.

7. Wundnadel (10) nach einem der vorhergehenden Ansprüche, bei welcher am Spitzenkörper (22) ein Spreitzfächer (38) vorgesehen ist.

8. Wundnadel (10) nach dem vorhergehenden Anspruch, bei welcher der Spreitzfächer (38) Spreitzlamellen (40) umfasst, vorzugsweise vier bis zehn Lamellen.

9. Wundnadel (10) nach einem der vorhergehenden Ansprüche, bei welcher im Nadelkörper (12) ein Aufnahmeraum (42) für den Spreitzfächer (38) vorgesehen ist.

10. Wundnadel (10) nach einem der vorhergehenden Ansprüche, bei welcher der Faden (20) mehrfasrig ist.

11. Wundnadel (10) nach Anspruch 7, bei welcher der Faden (20) geflochten oder gesponnen ist.

12. Wundnadel (10) nach einem der vorhergehenden Ansprüche, bei welcher sich die Spitze durch Zugkräfte von 1 N oder weniger von dem Nadelkörper (12) lösen lässt.

13. Verfahren zur medizinischen, schönheitschirugischen, veterinärmedizinischen oder kommerziellen Anwendung, welches folgende Schritte umfasst:
a. Setzen eines Einstichs mit der Nadel im ersten Wundgewebe,
b. Weiterführen der Nadel zum zweiten Wundgewebe, wobei die Nadel durch den Einstich im ersten Wundgewebe geführt wird,
c. Setzen eines Einstichs im zweiten Wundgewebe, wobei die Nadel weiterhin durch den Einstich im ersten Wundgewebe geführt wird,
d. Führen der Spitze aus dem zweiten Wundgewebe, wobei die Nadel weiterhin durch den Einstich im ersten Wundgewebe geführt wird und wobei der Weg der Spitze der Nadel in den vorangegangenen Schritten eine Einführrichtung und einen Einführpfad festlegt,
e. Lösen der Nadelspitze vom Nadelkörper (12) im Wesentlichen durch Ausüben von Zug entgegen der Einführrichtung,
f. Rückführen des Nadelkörpers (12) ohne die Spitze aus dem zweiten Wundgewebe und aus dem ersten Wundgewebe in umgekehrter Richtung entlang des Einführpfades, wobei der Faden (20) im Wundgewebe verbleibt.

14. Verfahren nach dem vorhergehenden Anspruch, bei dem beim Rückführen des Nadelkörpers (12) die Spitze nicht gehalten wird.

15. Verfahren nach dem vorhergehenden Anspruch, bei dem sich die Spitze mit einem Spreitzfächer (38) am ersten Wundgewebe oder am zweiten Wundgewebe oder an dem ersten oder dem zweiten Wundgewebe benachbartes Gewebe abstützt.
